(19) Europäisches Patentamt
European Patent Office
Office européen des brevets

(11) Veröffentlichungsnummer: **0 036 992**
**B1**

(12) # EUROPÄISCHE PATENTSCHRIFT

(45) Veröffentlichungstag der Patentschrift: **22.12.82**

(51) Int. Cl.³: **C 07 C 153/07,** A 61 K 31/265

(21) Anmeldenummer: **81101914.0**

(22) Anmeldetag: **14.03.81**

(54) **Verwendung von S-Benzyl-3-benzylthiopropionthioat in Arzneimitteln.**

(30) Priorität: **28.03.80 DE 3011999**

(43) Veröffentlichungstag der Anmeldung: **07.10.81 Patentblatt 81/40**

(45) Bekanntmachung des Hinweises auf die Patenterteilung: **22.12.82 Patentblatt 82/51**

(84) Benannte Vertragsstaaten: **AT BE CH DE FR GB IT LI NL**

(56) Entgegenhaltungen:
**JOURNAL OF THE CHEMICAL SOCIETY, (B), 1971, Seiten 565-566, London, G.B. B. WLADISLAW et al.: "Interaction between the carbonyl group and an alpha-sulphur atom. Infrared and ultraviolet spectra of some alpha-(alkylthio)thioesters"**

(73) Patentinhaber: **Troponwerke GmbH & Co. KG, Berliner Strasse 156, D-5000 Köln 80 (DE)**

(72) Erfinder: **Etschenberg, Eugen, Dr., Virchowstrasse 20, D-5000 Koeln 41 (DE)**
Erfinder: **Jacobi, Haireddin, Dr., Finkenweg 2, D-5672 Leichlingen (DE)**
Erfinder: **Opitz, Wolfgang, Dr., Holzbachtalstrasse 60, D-5063 Overath (DE)**

(74) Vertreter: **Dill, Erwin, Dr. et al, c/o BAYER AG Zentralbereich Patente Marken und Lizenzen Bayerwerk, D-5090 Leverkusen 1 (DE)**

**EP 0 036 992 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verwendung von S-Benzyl-3-benzylthiopropionthioat in Arzneimitteln

Die vorliegende Erfindung betrifft die Verwendung des bekannten S-Benzyl-3-benzylthiopropionthioates der Formel (I)

(I)

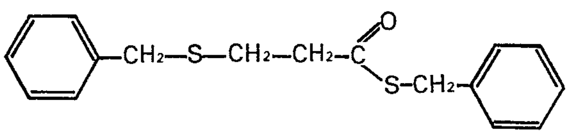

in Arzneimitteln, insbesondere in Arzneimitteln zur Behandlung entzündlicher Prozesse.

Die erfindungsgemässe Verbindung ist bereits als chemisches Individuum in der Literatur beschrieben (vgl. J. Chem. Soc. B. 1971, 565).

Es ist weiterhin bekannt, dass einige schwefelhaltige Carbonsäurederivate entzündungshemmende Wirkungen aufweisen (vgl. DE-OS 2753768, DE-OS 2824386).

Biologische bzw. pharmakologische Wirkungen der erfindungsgemässen Verbindung sind bisher nicht bekannt geworden. Überraschenderweise besitzt die erfindungsgemässe Verbindung bei guter Verträglichkeit sowohl bei intramuskulärer Applikation als auch bei oraler Verabreichung eine sehr starke entzündungshemmende Wirkung. Weiterhin ist sie im Vergleich zu den bekannten schwefelhaltigen Carbonsäurederivaten weniger geruchsintensiv und damit für pharmazeutische Zubereitungen und therapeutische Anwendung erheblich besser geeignet.

Die antiphlogistische Wirkung wurde nach oraler und i.m.-Applikation am Kaolinödem der Rattenpfote untersucht (Methode: Vinegar, R., R. Pharmac. Exp. Ther., 161, 389, 1968). Sowohl bei der parenteralen als auch bei der oralen Anwendung zeigt die erfindungsgemässe Verbindung bereits bei Dosierungen unter 10 mg/kg eine signifikante antiphlogistische Wirkung. Sie ist somit deutlich stärker wirksam als handelsübliche Antiphlogistika.

Die Untersuchung der akuten Toxizität zeigt, dass die Verbindung sowohl nach oraler als auch nach i.m.-Applikation an der Ratte sehr gut verträglich ist ($DL_{50}$ grösser als 1000 mg/kg).

Da bekanntlich viele Antiphlogistika zu Schäden an der Schleimhaut des Gastrointestinaltraktes führen können, wurde auch die ulcerogene Wirkung der Verbindung an der Ratte untersucht. Es zeigt sich, dass die erfindungsgemässe Verbindung auch hier eine bessere Verträglichkeit besitzt als bekannte Antiphlogistika.

Bei Kenntnis des Standes der Technik konnte nicht erwartet werden, dass das bekannte S-Benzyl-3-benzylthiopropionthioat eine so grosse Wirkungsstärke und eine so gute Verträglichkeit besitzt, dass diese Verbindung sowohl den chemisch ähnlichen schwefelhaltigen Carbonsäurederivaten als auch bekannten Wirkstoffen, die als Antiphlogistika eingesetzt werden, überlegen ist.

Die Herstellung der erfindungsgemässen Verbindung erfolgt nach bekannten Verfahren, vorzugsweise durch Umsetzung von Acrylsäurechlorid mit Benzylmercaptan und wässriger Natronlauge im molaren Verhältnis 1:2:1 in Gegenwart von inerten organischen Lösungsmitteln, insbesondere Dioxan, bei Temperaturen zwischen -10 und 40°C umsetzt.

Die Aufarbeitung des zweiphasigen Reaktionsgemisches erfolgt vorzugsweise durch Extraktion des Produktes mittels hydrophober organischer Lösungsmittel, insbesondere Dichlormethan mit anschliessender Destillation der erfindungsgemässen Verbindung.

Zur vorliegenden Erfindung gehören pharmazeutische Zubereitungen, die neben üblichen pharmazeutisch geeigneten Hilfs- und Trägerstoffen die erfindungsgemässe Verbindung enthalten, sowie Verfahren zur Herstellung dieser Zubereitungen.

Zur vorliegenden Erfindung gehören auch pharmazeutische Zubereitungen in Dosierungseinheiten. Dies bedeutet, dass die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder ½, ⅓ oder ¼ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter pharmazeutisch geeigneten Trägerstoffen sind übliche feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den Wirkstoff neben den üblichen Trägerstoffen enthalten, wie Füll- und Streckmittel (z.B. Stärken, Milchzucker, Rohrzucker, Glukose, Mannit und Kieselsäure), Bindemittel (z.B. Carboxymethylcellulose, Alginate, Gelatine, Polyvinylpyrrolidon), Feuchthaltemittel (z.B. Glycerin), Sprengmittel (z.B. Agar-Agar, Calciumcarbonat und Natriumbicarbonat), Lösungsverzögerer (z.B. Paraffin) und Resorptionsbeschleuniger (z.B. quarternäre Ammoniumverbindungen), Adsorptionsmittel (z.B. Kaolin und Bentonit) und Gleitmittel (z.B. Talkum, Calcium- und Magnesiumstearat und feste Polyethylenglykole) oder Gemische der aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der Wirkstoff kann gegebenenfalls mit einem oder mehreren der oben angegebenen Trägerstoffe auch in mikroverkapselter Form vorliegen zur Erzielung eines Retardeffektes.

Suppositorien können neben dem Wirkstoff die üblichen wasserlöslichen oder wasserunlöslichen Trägerstoffe enthalten, z.B. Polyethylenglykol, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem Wirkstoff die üblichen Trägerstoffe enthalten (z.B. tierische und pflanzliche Fette, Wachse, Paraffine, Stärken, Tragant) oder Gemische dieser Stoffe.

Puder und Sprays können neben dem Wirkstoff die üblichen Trägerstoffe enthalten (z.B. Milchzucker, Talkum, Kieselsäure, Aluminiumhydroxid) oder Gemische dieser Stoffe. Sprays können zusätzlich die üblichen Treibmittel enthalten.

Lösungen und Emulsionen können neben dem Wirkstoff die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, Öle, insbesondere Baumwollsaatöl, Erdnussöl, Maiskeimöl, Olivenöl und Sesamöl, Glycerin, Polyethylenglykole oder Gemische dieser Stoffe enthalten.

Zur parenteralen Applikation können die Lösungen und Emulsionen auch in steriler und blutisotonischer Form vorliegen.

Suspensionen können neben dem Wirkstoff die üblichen Trägerstoffe wie flüssige Verdünnungsmittel (z.B. Wasser, Ethylalkohol, Propylenglykol), Suspendiermittel (z.B. ethoxylierte Isostearylalkohole, Polyoxyethylensorbit- und Sorbitanester, mikrokristalline Cellulose) oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süssmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben ausgeführten pharmazeutischen Zubereitungen können ausser dem erfindungsgemässen Wirkstoff auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des Wirkstoffes mit dem oder den Trägerstoffen.

Zur vorliegenden Erfindung gehört auch die Verwendung des erfindungsgemässen Wirkstoffes sowie von pharmazeutischen Zubereitungen, die den erfindungsgemässen Wirkstoff enthalten, in der Human- und Veterinärmedizin zur Verhütung, Besserung und/oder Heilung entzündlicher Prozesse im menschlichen und tierischen Organismus.

Der Wirkstoff oder die pharmazeutischen Zubereitungen können oral, parenteral, intraperitoneal oder rectal, vorzugsweise intramuskulär oder oral appliziert werden.

Im allgemeinen ist es in der Humanmedizin als vorteilhaft anzusehen, den erfindungsgemässen Wirkstoff bei oraler Applikation in Gesamtmengen von etwa 100 bis 4000, vorzugsweise 200 bis 2000 mg/Person je 24 Stunden, gegebenenfalls in Form mehrerer Einzelgaben, zur Erzielung der gewünschten Ergebnisse zu verabreichen. Bei intramuskulärer Applikation kann der erfindungsgemässe Wirkstoff in Gesamtmengen von 10 bis 1500 mg/Person, insbesondere 50 bis 800 mg/Person je 24 Stunden, gegebenenfalls in Form mehrerer Einzelgaben, verabreicht werden. Eine Einzelgabe enthält den erfindungsgemässen Wirkstoff, vorzugsweise in Mengen von etwa 10 bis etwa 300, insbesondere 50 bis 200 mg/Dosis. Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation sowie dem Zeitraum bzw. Zeitintervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muss. Die Festlegung der jeweils erforderlichen optimalen Dosierung und Applikationsart des Wirkstoffes kann durch jeden Fachmann aufgrund seines Fachwissens leicht erfolgen.

Beispiele

Herstellung von S-Benzyl-3-benzylthiopropionthioat

Ein Gemisch aus 69 g (0,55 Mol) Benzylmercaptan, 10,8 g (0,27 Mol) NaOH in 216 ml Wasser und 100 ml Dioxan wird unter Eiskühlung und Rühren tropfenweise mit 22 ml (0,27 Mol) Acrylsäurechlorid versetzt. Das zweiphasige Reaktionsgemisch wird über Nacht bei Raumtemperatur gerührt, das Produkt mit Dichlormethan extrahiert und im Kugelrohr destilliert.

Ausbeute: 53,05 g (64,9% der Theorie) $Kp_{0,05}$ = 240-250° $^1$H-NMR ($CDCl_3$): $\delta$ ppm = 2.65 (S,2H); 3.62 (S,2H); 4.03 (S,2H); 7.21 (S,10H).

## Patentansprüche

1. S-Benzyl-3-benzylthiopropionthioat zur Verwendung bei der Behandlung entzündlicher Prozesse.

2. Arzneimittel zur Behandlung entzündlicher

Prozesse enthaltend S-Benzyl-3-benzylthiopropionthioat.

3. Verfahren zur Herstellung von Arzneimitteln, dadurch gekennzeichnet, dass man S-Benzyl-3-benzylthiopropionthioat als Wirkstoff gemäss Anspruch 1, gegebenenfalls unter Verwendung pharmazeutisch geeigneter Hilfs- und Trägerstoffe in eine geeignete Applikationsform überführt.

**Claims**

1. S-benzyl 3-benzylthiopropionothioate for use in the treatment of inflammatory processes.

2. Medicaments for the treatment of inflammatory processes, containing S-benzyl 3-benzylthiopropionothioate.

3. Process for the production of medicaments, characterised in that S-benzyl 3-benzylthiopropionothioate as the active compound according to Claim 1, is converted into a suitable form of administration, if appropriate using pharmaceutically suitable auxiliaries and excipients.

**Revendications**

1. 3-benzylthiopropionothioate de S-benzyle pour l'emploi dans le traitement de processus inflammatoires.

2. Médicament pour le traitement de processus inflammatoires contenant du 3-benzylthiopropionothioate de S-benzyle.

3. Procédé de préparation de médicaments, caractérisé en ce qu'on convertit du 3-benzylthiopropionothioate de S-benzyle comme matière active selon la revendication 1, éventuellement avec utilisation de substance auxiliaires et de support pharmaceutiquement appropriées, en une forme d'application appropriée.